(19) Europäisches Patentamt
European Patent Office
Office européen des brevets

(11) EP 4 183 394 A1

(12) EUROPEAN PATENT APPLICATION
published in accordance with Art. 153(4) EPC

(43) Date of publication:
24.05.2023 Bulletin 2023/21

(21) Application number: 21841781.4

(22) Date of filing: 15.07.2021

(51) International Patent Classification (IPC):
*A61K 31/4196* (2006.01) *A61P 31/14* (2006.01) *A61P 31/16* (2006.01)

(52) Cooperative Patent Classification (CPC):
A61K 31/4196

(86) International application number:
PCT/CN2021/106486

(87) International publication number:
WO 2022/012624 (20.01.2022 Gazette 2022/03)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 17.07.2020 CN 202010692337

(71) Applicant: Academy of Military Medical Sciences
Beijing 100850 (CN)

(72) Inventors:
- ZHONG, Wu
Beijing 100850 (CN)
- ZHOU, Xinbo
Beijing 100850 (CN)
- CAO, Ruiyuan
Beijing 100850 (CN)
- XIAO, Dian
Beijing 100850 (CN)
- WANG, Manli
Beijing 100850 (CN)
- FAN, Shiyong
Beijing 100850 (CN)
- HU, Zhihong
Beijing 100850 (CN)
- LI, Song
Beijing 100850 (CN)

(74) Representative: Inspicos P/S
Agern Allé 24
2970 Hørsholm (DK)

# (54) USE OF 2,4,5-TRISUBSTITUTED 1,2,4-TRIAZOLONE IN ANTIVIRAL

(57) Related is application of 2,4,5-trisubstituted 1,2,4-triazolones in antiviral therapy, particularly related to use of a compound of formula I in the preparation a medicament, wherein the medicament is used to prevent and/or treat a disease associated with a virus. It is shown in vitro experiment that, the compound effectively inhibits viral infections, and shows low toxicity in some tests,

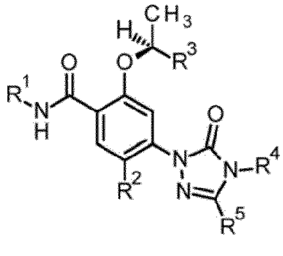

I.

EP 4 183 394 A1

## Description

The present application is based on and claims the benefit of priority to Chinese patent application 202010692337.9 filed July 17, 2020 which is hereby incorporated by reference in its entirety.

## Technical field

The present application relates to chemical drug field, particularly relates to application of 2,4,5-trisubstituted 1,2,4-triazolones in antiviral therapy. *In vitro* tests show that the compound can effectively inhibit various viral infections, especially the compound BAY 2402234 has effective inhibitory effect on Coronavirus, influenza virus, enterovirus, zika virus, bunya virus, etc..

## Background art

Virus is a non-cellular life that only has a nucleic acid (DNA or RNA), and must parasitize in living cells and multiply by replication. It infects the organism in many ways, and replicates in susceptible host cells.

Viruses infecting humans can cause varying degrees of damage to the human body. For example, some diseases caused by viruses are almost fatal, such as HIV, Ebola virus, rabies virus, etc.; other diseases caused by viruses will cause people to lose labor and even life-long disability, such as hepatitis caused by hepatitis A, hepatitis B and other 5 types of hepatitis virus, viral myocarditis caused by coxsackie virus, influenza virus, etc.. In addition, other viral diseases also pose serious threats to human health, such as dengue virus and zika virus of Flaviviridae, bunya virus of Bunyaviridae, enterovirus of Picornaviridae, etc..

Among them, Coronavirus is an enveloped, unsegmented, single-stranded positive-stranded RNA virus that has a wide range of animal hosts. Coronavirus like SARS and MERS derived from animal infectious diseases can cause death in humans.

On February 11, 2020, the International Committee on Taxonomy Viruses (ICTV) announced a new coronavirus -- severe acute respiratory syndrome coronavirus 2 (SARS-CoV-2). On the same day, the World Health Organization (WHO) announced that the official name of the disease caused by this virus is COVID-19. At present, the novel coronavirus infection is mainly treated with supportive therapy in clinic, and no specific antiviral drug is available. It is urgent and necessary to develop drugs that can effectively inhibit the above viruses, especially SARS-CoV-2.

Compounds of 2,4,5-trisubstituted 1,2,4-triazolones (formula I as shown below) are novel antitumor drugs developed by Bayer AG. The antitumor activity is exerted by inhibiting proliferation of tumor cells and inducing differentiation. The drugs have great clinical treatment potential for bone marrow malignant tumors. Among those compounds, BAY 2402234 shows good antitumor activity both *in vivo* and *in vitro,* and is currently undergoing phase I clinical trial research with indications for bone marrow tumors.

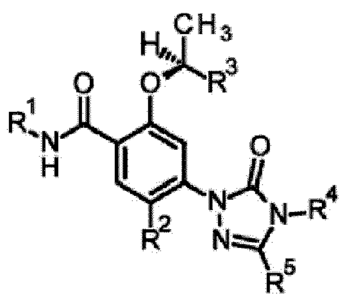

I

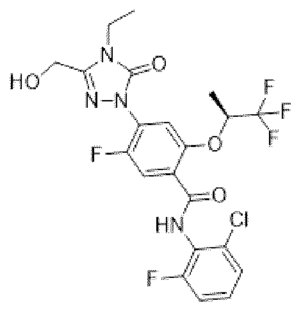

BAY 2402234

## Content of the invention

In the research, the inventor found that BAY 2402234 could inhibit various viruses, and showed low cytotoxicity in some tests. This provides a new way and option for effectively preventing and/or treating diseases or symptoms caused by viruses.

In a first aspect, the disclosure provides use of a compound of formula I, or its N-oxide, tautomer, geometric isomer, solvate, hydrate, pharmaceutically acceptable salt, or pharmaceutically acceptable salt of the tautomer or N-oxide thereof in the manufacture of a medicament, wherein the medicament is used in preventing and/or treating a disease associated with a virus, and the compound of formula I is as shown below,

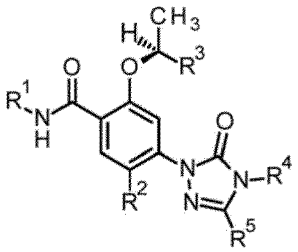

I

wherein,

$R^1$ represents a group selected from

3-pentyl, 2,2-dimethylpropyl, 4-heptyl, 4-fluorophenylcyclopropyl, cyclopentyl, cyclohexyl, cycloheptyl, cyclopentylmethyl, cyclohexylmethyl, 1-cyclohexylethyl, 1-hydroxypropan-2-yl, 2-hydroxypropyl, 1-hydroxybutan-2-yl, 1-cyanobutan-2-yl, 1-phenylbutan-2-yl, 1-amino-2-propyl, 1-amino-2-butyl, 1-amino-1-oxobutan-2-yl, indan-2-yl,

a 5- to 6-membered heterocycloalkyl group, which is selected from tetrahydrofuran-3-yl, tetrahydro-2H-pyran-4-yl and piperidin-4-yl, and which is optionally substituted one or two times with a methyl group,

a phenyl group, which is optionally substituted, one, two or three times, each substituent is independently selected from a fluorine atom or a chlorine atom or a group selected from methyl, ethyl, propyl, isopropyl, difluoromethyl, trifluoromethyl, methoxy, -O-C(=O)-1,1-dimethylethyl, hydroxy, $-C(=O)OCH_3$, -C(=O)NH-cyclopropyl, amino, methylamino, aminomethyl, $-S-CH_3$, $-S(=O)_2CH_3$, and $-S(=O)(NH)CH_3$, and

a monocyclic heteroaryl group, which is selected from oxazol-2-yl, pyrazol-3-yl, pyrazol-5-yl, pyridin-2-yl, pyridin-3-yl, pyridin-4-yl, pyrimidin-2-yl, pyrimidin-4-yl, chinolin-5-yl, indazol-5-yl, and which is optionally substituted one or two times, each substituent is independently selected from methyl and methoxy,

$R^2$ represents a hydrogen atom or a fluorine or chlorine atom,

$R^3$ represents a group selected from propyl, 2-methylpropyl, 3-pentyl, cyclopropylmethyl, cyclopropyl, cyclopropylmethyl, cyclobutyl, cyclopentyl, cyclohexyl, difluoromethyl, trifluoromethyl, 1,1-difluoroethyl, prop-2-en-1-yl, 2-methyl-prop-1-en-1-yl, N,N-dimethylaminoethyl, and phenyl,

$R^4$ represents a group selected from methyl, ethyl, propyl, isopropyl, 2-butyl, prop-2-en-1-yl, cyclopropylmethyl, benzyl, cyclopropyl, cyclobutyl, cyclopentyl, and 2-hydroxyethyl,

$R^5$ represents a chlorine atom or a group selected from methyl, ethyl, propyl, isopropyl, 2-butyl, isobutyl, tert-butyl, cyclopropyl, cyclobutyl, cyclopentyl, trifluoromethyl, hydroxymethyl, 1-hydroxy ethyl, 2-hydroxypropan-2-yl, 1-chloroethyl, 1-hydroxy-2,2,2-trifluoroethyl, 1-methoxyethyl, methoxy, isopropoxy, methylsulfanyl, aminomethyl, (methylamino)methyl, (dimethylamino)methyl, 1-aminoethyl, 2-aminoethyl, methylamino and ethyl(methyl)amino, -C(=O)OH, $-C(=O)OCH_3$, $-C(=O)NH_2$, $-C(=O)NHCH_3$, -C(=O)NHcyclopropyl, $-C(=O)N(CH_3)_2$, and $-S(=O)(=NH)CH_3$.

In some embodiments, the compound is the compound of formula II,

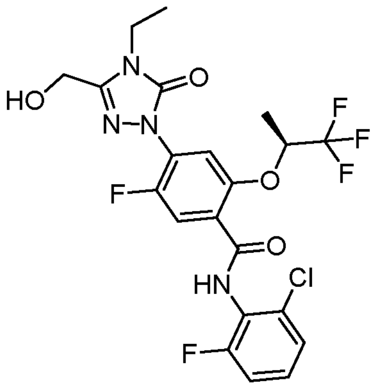

II.

In some embodiments, the virus is a RNA virus.

In some embodiments, the virus is a Coronaviridae virus, an Orthomyxoviridae virus, a Flaviviridae virus, a Bunyaviridae virus, a Picornaviridae virus, an Arenavirus, a Filoviridae virus, or a WEE virus.

In some embodiments, the virus is a Coronaviridae virus.

In some embodiments, the Coronaviridae virus is selected from HCoV-229E, HCoV-OC43, HCoV-NL63, HCoV-HKU1, SARS-CoV, MERS-CoV, and SARS-CoV-2.

In some embodiments, the virus is SARS-CoV-2.

In some embodiments, the virus is an Orthomyxoviridae virus.

In some embodiments, the virus is an influenza virus.

In some embodiments, the Orthomyxoviridae virus is an influenza virus.

In some embodiments, the influenza virus is Influenza A virus (such as H1N1, H5N1, H7N1, H7N2, H7N3, H7N7, H7N9, H9N2, or H10N8), Influenza B virus, or Influenza C virus.

In some embodiments, the virus is a Flaviviridae virus.

In some embodiments, the Flaviviridae virus is selected from Zika virus, Dengue virus, West Nile virus, Yellow fever virus and HCV.

In some embodiments, the virus is a Bunyaviridae virus.

In some embodiments, the Bunyaviridae virus is Bunya virus or Phlebovirus.

In some embodiments, the virus is a Picornaviridae virus.

In some embodiments, the Picornaviridae virus is Enterovirus or Foot-and-Mouth disease virus.

In some embodiments, the virus is a Filoviridae virus.

In some embodiments, the Filoviridae virus is selected from Ebola virus, Marburg virus, and Cueva virus.

In some embodiments, the medicament is used to prevent and/or treat a disease caused by SARS-CoV, MERS-CoV, SARS-CoV-2, Influenza virus, Zika virus, Dengue virus, Bunya virus, or Enterovirus. In some preferred embodiments, the medicament is used to prevent and/or treat a disease caused by SARS-CoV, MERS-CoV, or SARS-CoV-2, for example, SARS, MERS or COVID-19.

BAY 2402234 has excellent antivirus activity against Coronavirus, especially SARS-CoV-2, and can be used to treat diseases caused by infecting the virus, for example, simple infection, such as fever, cough, and pharyngalgia etc., pneumonia, acute or severe respiratory infection, hypoxic respiratory failure, acute respiratory distress syndrome, sepsis, and septic shock etc.. Through creative research, the inventor found that BAY 2402234 can inhibit the replication of SARS-CoV-2, and has excellent therapeutic effect on diseases caused by SARS-CoV-2. Thus, in some particularly preferred embodiments, the medicament is used to prevent and/or treat a disease caused by SARS-CoV-2, such as COVID-19.

In some embodiments, the medicament is for human use.

In some embodiments, the medicament is for animal use.

In some embodiments, the medicament further comprises a pharmaceutically acceptable carrier or auxiliary.

In some embodiments, the compound is the only pharmaceutical active ingredient.

In some embodiments, the compound is used in combination with other pharmaceutical active ingredients. In some embodiments, the compound and the other pharmaceutical active ingredients are in the same preparation unit. In some embodiments, the compound and the other pharmaceutical active ingredients are in different preparation units. In some embodiments, the compound and the other pharmaceutical active ingredients are administered concurrently, separately or successively. In some embodiments, the other pharmaceutical active ingredients are antiviral drugs, such as amantadine, rimantadine, enfuvirtide, maraviroc, acyclovir, ganciclovir, valaciclovir, famciclovir, sodium phosphonate,

lamivudine, zidovudine, enteltabine, tenofovir, adefovir dipivoxil, efavirenz, nevirapine, saquinavir, oseltamivir, zanamivir, ribavirin, and interferon etc..

In some embodiments, the medicament is a solid preparation or a liquid preparation. In some embodiments, the medicament is a tablet, an injection, or a spray. In some embodiments, the medicament is a tablet or injection.

In a second aspect, the disclosure also relates to use of the compound as defined in the first aspect, or its N-oxide, tautomer, geometric isomer, solvate, hydrate, pharmaceutically acceptable salt, or pharmaceutically acceptable salt of the tautomer or N-oxide thereof in the manufacture of a medicament, wherein the medicament is used to inhibit the replication or reproduction of a virus in a cell (e.g., a mammal cell).

In some embodiments, the animal is human, dog or pig.

In some embodiments, the medicament is for human use or animal use.

In some embodiments, the medicament further comprises a pharmaceutically acceptable carrier or auxiliary.

In some embodiments, the compound is the only pharmaceutical active ingredient or used in combination with other pharmaceutical active ingredients (e.g., the medicament is a compound preparation).

In some embodiments, the other pharmaceutical active ingredients are antiviral drugs, such as one or more selected from amantadine, rimantadine, enfuvirtide, maraviroc, acyclovir, ganciclovir, valaciclovir, famciclovir, sodium phosphonate, lamivudine, zidovudine, enteltabine, tenofovir, adefovir dipivoxil, efavirenz, nevirapine, saquinavir, oseltamivir, zanamivir, ribavirin, and interferon etc..

In some embodiments, the medicament is a solid preparation or a liquid preparation.

In some embodiments, the medicament is a tablet, an injection, or a spray, preferably a tablet or injection.

In a third aspect, the disclosure also relates to use of the compound as defined in the first aspect, or its N-oxide, tautomer, geometric isomer, solvate, hydrate, pharmaceutically acceptable salt, or pharmaceutically acceptable salt of the tautomer or N-oxide thereof in the manufacture of a virus inhibitor.

In some embodiments, the inhibitor is used to inhibit the replication or reproduction of a virus in a cell (e.g., a mammal cell).

In some embodiments, the host is a mammal.

In some embodiments, the mammal is human, dog or pig.

In a fourth aspect, the disclosure relates to a method of preventing and/or treating a disease associated with a virus, comprising a step of administering to a subject in need an effective amount of the compound as defined in the first aspect, or its N-oxide, tautomer, geometric isomer, solvate, hydrate, pharmaceutically acceptable salt, or pharmaceutically acceptable salt of the tautomer or N-oxide thereof.

In some embodiments, the subject is a mammal, such as human.

In some embodiments, the compound, or its N-oxide, tautomer, geometric isomer, solvate, hydrate, pharmaceutically acceptable salt, or pharmaceutically acceptable salt of the tautomer or N-oxide thereof is administered alone, or in combination with other pharmaceutical ingredient, for example, administered concurrently, separately or successively.

In some embodiments, the other pharmaceutical active ingredients are antiviral drugs, such as one or more selected from amantadine, rimantadine, enfuvirtide, maraviroc, acyclovir, ganciclovir, valaciclovir, famciclovir, sodium phosphonate, lamivudine, zidovudine, enteltabine, tenofovir, adefovir dipivoxil, efavirenz, nevirapine, saquinavir, oseltamivir, zanamivir, ribavirin, and interferon etc..

In a fifth aspect, the disclosure also relates to a method of inhibiting the replication or reproduction of a virus in a cell (e.g., a mammal cell), comprising a step of administering to a subject in need or contacting the cell with an effective amount of the compound as defined in the first aspect, or its N-oxide, tautomer, geometric isomer, solvate, hydrate, pharmaceutically acceptable salt, or pharmaceutically acceptable salt of the tautomer or N-oxide thereof.

In some embodiments, the subject is a mammal, such as human.

In some embodiments, the mammal cell is from human cell.

In some embodiments, the compound, or its N-oxide, tautomer, geometric isomer, solvate, hydrate, pharmaceutically acceptable salt, or pharmaceutically acceptable salt of the tautomer or N-oxide thereof is administered alone, or in combination with other pharmaceutical ingredient, for example, administered concurrently, separately or successively.

In some embodiments, the other pharmaceutical active ingredients are antiviral drugs, such as one or more selected from amantadine, rimantadine, enfuvirtide, maraviroc, acyclovir, ganciclovir, valaciclovir, famciclovir, sodium phosphonate, lamivudine, zidovudine, enteltabine, tenofovir, adefovir dipivoxil, efavirenz, nevirapine, saquinavir, oseltamivir, zanamivir, ribavirin, and interferon etc..

The virus and disease in the second to the fifth aspects is as defined in the first aspect.

In some embodiments, the virus is a Coronavirus, such as HCoV-229E, HCoV-OC43, HCoV-NL63, HCoV-HKU1, SARS-CoV, MERS-CoV, or SARS-CoV-2, especially SARS-CoV-2.

In some embodiments, the disease is a disease caused by SARS-CoV-2, for example, simple infection (such as fever, cough, and/or pharyngalgia), pneumonia, acute or severe respiratory infection, hypoxic respiratory failure,

acute respiratory distress syndrome, sepsis, or septic shock, particularly COVID-19.

In the present application, unless otherwise indicated, the scientific and technical terms used herein have the meanings commonly understood by those skilled in the art. Also, the procedures of cell culture, molecular genetics, nucleic acid chemistry, immunology laboratory operation used herein are all conventional and widely used in the corresponding fields. Meanwhile, for the purpose of better understanding of the present invention, the definitions and explanations of related terms are provided below.

As used herein, the term "pharmaceutically acceptable salt" includes an inorganic acid salt or an organic acid salt, and an inorganic base salt or an organic base salt, such as sodium salts, potassium salts, calcium salts, lithium salts, meglumine salt, hydrochloride salts, hydrobromide salts, hydroiodide salts, nitrates, sulfates, phosphates, hydrogen phosphates, acetates, propionates, butyrates, oxalates, trimethyl acetates, adipates, alginates, lactates, citrates, tartrates, succinates, maleates, fumarates, picrates, aspartates, gluconates, benzoates, methanesulfonates, ethanesulfonates, benzenesulfonates, p-toluenesulfonates or pamoates, etc..

As used herein, the term "geometric isomer" refers to a stereoisomer resulted from different spatial arrangements in molecules having a double bond or ring structure due to the hindrance of the free rotation of atoms or groups of atoms connected to the double bond or ring, such as cis/trans isomers.

A compound of formula I in the disclosure may exist in the form of a solvate (preferably a hydrate), and it comprises a polar solvent, especially water, methanol or ethanol, as a structural element of the lattice. The amount of the polar solvent is stoichiometrical or non-stoichiometrical. It shall be understood that, even though the solvates of the compound of formula I used in the treatment of the disease or infection defined in the present application may have different properties (including pharmacokinetic properties), once absorbed in the subject, the compound of formula I will be obtained, so that the use of the compound of formula I covers the use of any solvate of the compound of formula I.

Those skilled in the art should understand that since nitrogen requires available lone pairs of electrons to be oxidized into oxides, not all nitrogen-containing heterocycles can form N-oxides; those skilled in the art will recognize the nitrogen-containing heterocycles can form N-oxides. Those skilled in the art will also recognize that tertiary amines can form N-oxides. The synthetic methods used to prepare N-oxides of heterocycles and tertiary amines are well known to those skilled in the art, including the use of peroxyacids, such as peracetic acid, m-chloroperoxybenzoic acid (MCPBA), hydrogen peroxide, alkyl hydroperoxides such as tert-butyl hydroperoxide, sodium perborate, and dioxirane (dioxirane) such as dimethyl bis ethylene oxide, to oxidize heterocycles and tertiary amines. These methods for preparing N-oxides have been widely described and reviewed in the literature, see for example: T. L. Gilchrist, Comprehensive Organic Synthesis, vol. 7, pp 748-750; A. R. Katritzky and A. J. Boulton, Eds., Academic Press; as well as G. W. H. Cheeseman and E. S. G. Werstiuk, Advances in Heterocyclic Chemistry, vol. 22, pp 390-392, A. R. Katritzky and A. J. Boulton, Eds., Academic Press.

The compound of formula I may exist in a mixture of two or more structurally different forms in rapid equilibrium (usually referred to as tautomers). Representative examples of tautomers include keto-enol tautomers, phenol-ketone tautomers, nitroso-oxime tautomers, imine-enamine tautomers, etc.. It is to be understood that the scope of the application covers all such isomers in any ratio (e.g. 60%, 65%, 70%, 75%, 80%, 85%, 90%, 95%, 96%, 97%, 98%, 99%) or mixtures thereof.

In this application, the term "therapeutically effective amount" or "prophylaxically effective amount" refers to, within the scope of reasonable medical judgment, an amount sufficient to treat or prevent the patient's disease but low enough to avoid serious side effects (at a reasonable benefit/risk ratio). The therapeutically effective amount of the compound may vary based on factors including the specific compound selected (for example, considering the potency, effectiveness and half-life of the compound), the selected route of administration, the disease to be treated, the severity of the disease to be treated, age, size, weight, and physical disease of the patient being treated, the medical history of the patient being treated, the duration of treatment, the nature of concurrent therapy, the desired therapeutic effect, and etc., but it can still be routinely determined by those skilled in the art.

In addition, it should be pointed out that the specific dosage and route of administration of the compound of formula I, or a geometric isomer, a pharmaceutically acceptable salt, a solvate or a hydrate thereof are determined by many factors, including the age of the patient, body weight, gender, health status, nutritional status, potency of the drug, time taken, metabolic rate, severity of the disease, and the subjective judgment of the physician. Preferable dose is between 0.001-1000 mg/kg body weight/day.

## Description of the drawings

The description of the drawings herein is provided for further explanation of the present invention, and constitutes part of the present application. The exemplary embodiments and description are meant to explain the present invention, and should not be understood as any inappropriate limitation to the present invention. In the drawings:

Figure 1 shows the effect on viral RNA load in Vero E6 cells infected by SARS-CoV-2, wherein (a) shows BAY

2402234 can inhibit the viral RNA load in the cells 24h after the cells were infected by SARS-CoV-2, and the inhibitory activity is dose-dependent; the left ordinate is the percentage inhibition rate (corresponding to the red dots and fitting curve therefrom) calculated from copy number of viral RNA in the sample, and the right ordinate is the percentage cytotoxicity calculated from cell viability (corresponding to the blue dots and fitting curve therefrom), the abscissa is the drug concentration; (b) shows that BAY 2402234 can inhibit the viral RNA load in the cells 48h after the cells were infected by SARS-CoV-2, and the inhibitory activity is dose-dependent; the left ordinate is the percentage inhibition rate (corresponding to the red dots and fitting curve therefrom) calculated from copy number of viral RNA in the sample, and the right ordinate is the percentage cytotoxicity calculated from cell viability (corresponding to the blue dots and fitting curve therefrom), the abscissa is the drug concentration.

Figure 2 shows the cytotoxicity of BAY 2402234 and its *in vitro* anti-influenza virus activity.

## Specific mode for carrying out the invention

The present invention is further illustrated clearly and completely in the following examples in conjunction with the drawings. Obviously, they are merely part, and not all of the examples. At least one of the following examples are illustrative and should not be understood as any limitation to the present invention, as well as its application and use. And other embodiments made by a person skilled in the art without creative work in light of the present invention all fall within the protection scope of the present invention.

Example 1: Experiment of BAY 2402234 in reduction of viral nucleic acid load of cells infected by SARS-CoV-2

(1) Drug treatment of virus-infected cells

Vero E6 cells (purchased from ATCC, Catalog No. 1586) were placed into a 24-well plate and incubated for 24 hours, then virus infection was carried out, specifically, SARS-CoV-2 (2019-nCoV) virus (nCoV-2019BetaCoV/Wuhan/WIV04/2019 strain, provided by Wuhan Institute of Virology, Chinese Academy of Sciences) was diluted with 2% cell maintenance solution (formulation: FBS (purchased from Gibco, Catalog No.: 16000044) was added to MEM (purchased from Gibco, Article No: 10370021) by a volume ratio of 2%, thereby obtaining the 2% cell maintenance solution) to corresponding concentration, and then added to the 24-well plate so that each well contained a viral load of 100$TCID_{50}$. Next, BAY 2402234 (purchased from MCE, Article No.: HY-112645) was diluted with 2% cell maintenance solution to the corresponding concentrations and added to corresponding wells, so that the final drug concentrations were 100 μM, 33 μM, 11 μM, 3.7 μM, 1.23 μM, 0.41 μM, 0.14 μM, respectively, then the plate was put in 37°C, 5% $CO_2$ incubator and continuously cultured for 48h, and the cell vehicle control group was added with only 2% cell maintenance solution without any test drug.

(2) RNA extraction

The RNA extraction kit was purchased from Qiagen, Article No.: 74106. The consumptive materials (spin column, RNase-free 2ml collection tube, etc.) and reagents (RLT, RW1, RPE, RNase-free water, etc.) involved in the following RNA extraction steps were all parts of the kit. The following extraction steps were all recommended by the kit instructions.

1) 100 μL of the supernatant was taken from the test plate, added to a nuclease-free EP tube, then added with 350 μL of Buffer RLT, mixed by a transfer liquid gun to make it fully lysed, and centrifuged to take the supernatant;

2) the supernatant obtained in step 1) was added with an equal volume of 70% ethanol and mixed well;

3) the mixed solution obtained in step 2) above was transferred to a RNase-free spin column, centrifuged at 12000 rpm for 15s, and the waste liquid was discarded;

4) 700μL of Buffer RW1 was added to the spin column, then centrifugation was carried out at 12000 rpm for 15s to clean the spin column, and the waste liquid was discarded;

5) 500μL of Buffer RPE was added to the spin column, then centrifugation was carried out at 12000 rpm for 15s to clean the spin column, and the waste liquid was discarded;

6) 500μL of Buffer RPE was added to the spin column, then centrifugation was carried out at 12000 rpm for 2min

to clean the spin column, and the waste liquid was discarded;

7) the spin column was placed in a new RNase-free 2 ml collection tube, and centrifugation was carried out at 12000 rpm for 1 min to dry the spin column, and then the entire spin column was transferred to the 1.5 ml collection tube of step 8);

8) the spin column dried in step 7) was placed in a new 1.5ml collection tube, added with 30μl of RNase-free water, and centrifuged at 12000 rpm for 2min, the obtained eluent contained the corresponding RNA, and was added with RNase inhibitor (purchased from NEB, Article No.: M0314L), and detected with Nano Drop (purchased from Thermo scientific, Nano Drop One) to determine each RNA concentration.

(3) RNA reverse transcription

In the experiment, the reverse transcription kit (PrimeScript™ RT reagent Kit with gDNA Eraser, Catalog No. RR047Q) produced by TaKaRa Company was used for RNA reverse transcription. The steps were as follows.

1) gDNA removal: RNA samples from each experimental group were collected, and 1 μg thereof was taken and subjected to reverse transcription. First, 2 μl of 5× gDNA Eraser Buffer was added to the RNA sample of each experimental group, the reaction system was supplemented with RNase Free water to 10 μl, mixed well, and subjected to 42 °C water bath for 2 min to remove the gDNA that might exist in the sample;

2) Reverse transcription: the sample obtained in 1) was added with appropriate amounts of enzyme, primer Mix and reaction buffer, supplemented with RNase Free water to an volume of 20 μl, reacted under 37°C water bath for 15 min, then put in 85°C water bath for 5 sec, thereby obtaining cDNA via transcription.

(4) Real-time PCR

Fluorescence quantitative PCR was used to detect the copy number per ml of the original virus solution.

The reaction system was mixed using TB Green Premix (Takara, Cat#RR820A), and the amplification reaction and reading were carried out with StepOne Plus Real-time PCR instrument (brand: ABI). The copy number contained in per ml of the original virus solution was calculated. The steps were as follows:

1) Establishment of standards: the plasmid pMT-RBD (the plasmid was provided by Wuhan Institute of Virology, Chinese Academy of Sciences) was diluted to $5\times10^8$ copies/μL, $5\times10^7$ copies/μL, $5\times10^6$ copies/μL, $5\times10^5$ copies/μL, $5\times10^4$ copies/μL, $5\times10^3$ copies/μL, $5\times10^2$ copies/μL. 2 μL standard or cDNA template was taken for qPCR reaction.

2) The sequences of primers used in the experiment were as follows (all indicated in 5'-3' direction):

RBD-qF: CAATGGTTTAACAGGCACAGG

RBD-qR: CTCAAGTGTCTGTGGATCACG

3) The reaction procedure was as follows:

Pre-denaturation: 95 °C for 5 minutes;

Cycle parameters: 95 °C for 15 seconds, 54 °C for 15 seconds, 72 °C for 30 seconds, for a total of 40 cycles.

Inhibition rate (%) = (the copy number of RNA of the drug treatment group)/(the copy number of RNA of the infection group) × 100%

(5) Cytotoxicity test of drug

The detection of the drug cytotoxicity was performed using CCK-8 kit (Beoytime). Specific steps were as follows:

1) $1\times10^4$ Vero E6 (ATCC) cells were placed in a 96-well plate and incubated at 37°C for 8 hours.

2) The drug was diluted with DMSO to an appropriate concentration of mother liquor, and then diluted with MEM medium (purchased from Gibco, Catalog No. 10370021) containing 2% FBS (purchased from Gibco, Catalog No. 16000044) to the same concentration as that for the drug treatment. The original medium in the 96-well plate was discarded, 100 $\mu$L of drug-containing MEM medium was added to the cells, and three replicate wells were prepared for each concentration. Vehicle control (DMSO and medium were added to the cell wells, without adding drug) and blank control (DMSO and medium were added to the wells, without cells) were set up. After the drug was added, the cells were cultured at 37°C for 48 hours.

3) 20 $\mu$L of CCK-8 solution (Beoytime) was added to the well to be tested, mixed gently, without generating bubbles, and continuously incubated at 37 °C for 2 hours. $OD_{450}$ was read on a microplate reader (purchased from Molecular Devices, Model: SpectraMax M5), and cell viability was calculated:

$$\text{Cell activity (\%)} = (A_{(\text{drug treatment group})} - A_{(\text{blank control})}) / (A_{(\text{vehicle control})} - A_{(\text{blank control})}) \times 100\%$$

wherein A was the reading of the microplate reader.

(6) Experimental results

The results of the virus proliferation inhibition experiment showed that the test compound at concentrations of 10 $\mu$M, 3.3 $\mu$M, 1.1 $\mu$M, 0.3 $\mu$M, 0.1 $\mu$M and 0.03 $\mu$M could effectively inhibit the replication of the SARS-CoV-2 virus genome in the infected supernatant (Figure 1).

The cytotoxicity test results showed that the treatment of the test compound (BAY 2402234) did not change the cell viability at below 10 $\mu$M, that was, the test compound had no toxic effect on the cells at all test concentrations (Figure 1).

(7) Conclusion

The compound BAY 2402234 significantly inhibits SARS-CoV-2 isolates nCoV-2019BetaCoV/Wuhan/WIV04/2019, $EC_{50}$ of the compound is 0.0070 $\mu$M and 0.0027 $\mu$M respectively, 24 hours and 48 hours after the infection, $CC_{50}$ is 198.8$\mu$M, and corresponding therapeutic index SI is 28400 and 73629.63 respectively.

Example 2: *In vitro* evaluations of anti-influenza virus activity and safety of BAY 2402234

(1) Evaluation of anti-influenza virus activity of the drug

1) MDCK cells (purchased from ATCC, Catalog No.: CCL-34) were inoculated into a 96 wells plate at a concentration of $1.5\times10^4$ cells/well, wherein the medium is DMEM (purchased from Gibco, Catalog No.: 11995065) containing 10%FBS (purchased from Gibco, Catalog No.: 16000044), and cultured for 24 hrs at 37°C in a $CO_2$ incubator.

2) Before the experiment, the cells were wahsed 3 times with PBS (purchased from Gibco, Catalog No.: 10010049), 100$\mu$L of D/F-12 medium (purchased from Gibco, Catalog No.:11330032) containing 2$\mu$g/mL TPCK (purchased from Sigma, Catalog No.:T1426) was added. The 50mM BAY 2402234 (purchased from MCE, Catalog No.: HY-112645) mother liquor was then diluted with the above D/F-12 medium in a serial manner to 0.444 $\mu$M, 0.148 $\mu$M, 0.049 $\mu$M, 0.016 $\mu$M, 0.005 $\mu$M, 1.829 nM, 0.609 nM, and 0.203 nM, 50 $\mu$L of each was added to the cell culture plate. Finally, the influenza viruses A/PR/8 (preserved in Academy of Military Medical Sciences), A/California/07/2009 (preserved in Academy of Military Medical Sciences), A/Hongkong/08/1968 (purchased from ATCC, Catalog No.: VR-1679), A/Zhenxing1109/2010 (preserved in Academy of Military Medical Sciences), B/Lee/40 (purchased from ATCC, Catalog No.: VR-1535) were diluted with D/F-12 medium containing 2$\mu$g/mL TPCK to corresponding concentration, 50$\mu$L of the dilution was added into the 96-well plate to reach 100 $TCID_{50}$ for each well. The final concentration of the drug was 0.25 time to that of the drug before being treated, that is, the drug with 0.111 $\mu$M as initial concentration was diluted in 3-fold serial manner to reach final concentration of 0.111 $\mu$M, 0.037 $\mu$M, 0.012 $\mu$M, 0.004 $\mu$M, 0.0014 $\mu$M, 0.457 nM, 0.152 nM, and 0.051 nM. Negative control (wells only added with DMSO and the culture medium, without the drug) and Positive control (wells added with DMSO, culture medium and the virus, without the drug) were set up. The cells were cultured at 37°C for 72 hrs.

3) Buffer and substrate of CellTiter-Glo® Luminescent Cell Viability Assay Kit (purchased from Promega, Catalog No.: G7573) were mixed in dark as the work solution. The work solution and PBS were mixed in a ratio of 4:6. The liquid in the cell culture plate was discarded, and then 100μl of test reagent was added for each well, the 96-well plate was shaked in an Orbital oscillator for 7min to induce cell lysis. After stablizing the signal in dark for 5min, chemiluminescence was determiend by an enzyme-labeled instrument (purchased from Molecular Devices, SpectraMax M5), the plate reading program was the CellTiter-Glo preset program, and the cell viability was calculated:

$$\text{cell viability (\%)} = (A_{(\text{drug treatment group})} - A_{(\text{Positive control group})}) / (A_{(\text{Negative control group})} - A_{(\text{Positive control group})}) \times 100\%$$

wherein A was the reads from the enzyme-labeled instrument.

(2) Cytotoxicity test of the drug

1) MDCK cell (purchased from ATCC, Catalog: CCL-34) was inoculated into a 96-well plate at a concentration of $1.5\times10^4$ cell/well, wherein the medium is DMEM (purchased from Gibco, Catalog No.: 11995065) containing 10%FBS (purchased from Gibco, Catalog No.: 16000044), and cultured for 24 hrs at 37°C in a COz incubator.

2) Before the experiment, the cells were wahsed 3 times with PBS (purchased from Gibco, Catalog No.: 10010049), 150 μL of D/F-12 medium (purchased from Gibco, Catalog No.: 11330032) containing 2ug/mL TPCK (purchased from Sigma, Catalog No.:T1426) was added. And then 50mM BAY 2402234 (purchased from MCE, Catalog No.: HY-112645) mother liquor was then diluted with the above D/F-12 medium in a serial manner to 4 μM, 1.333 μM, 0.444 μM, 0.148 μM, 0.049 μM, 0.016 μM, 0.005 μM, 1.829 nM, 0.609 nM, 0.203 nM, 50 μL of each was added to the cell culture plate. The final concentration of the drug was 0.25 time to that of the drug before being treated, that is, the drug with 1μM as initial concentration was diluted in 3-fold serial manner to reach final concentration of 1 μM, 0.333 μM, 0.111 μM, 0.037 μM, 0.012 μM, 0.004 μM, 0.0014 μM, 0.457 nM, 0.152 nM, 0.051 nM. Negative control group (wells only added with DMSO and the culture medium without the drug) was set up. The cells were cultured at 37°C for 72 hrs.

3) Buffer and substrate of CellTiter-Glo® Luminescent Cell Viability Assay Kit (purchased from Promega, Catalog No.: G7573) were mixed in dark as the work solution. The work solution and PBS were mixed in a ratio of 4:6. The liquid in the cell culture plate was discarded, and then 100μl of test reagent was added for each well, the 96-well plate was shaked in an Orbital oscillator for 7min to induce cell lysis. After stablizing the signal in dark for 5min, chemiluminescence was determiend by an enzyme-labeled instrument (purchased from Molecular Devices, SpectraMax M5), the plate reading program was the CellTiter-Glo preset program, and the cytotoxicity was calculated:

$$\text{cytotoxicity (\%)} = (A_{(\text{Negative control group})} - A_{(\text{Drug treatment group})}) / A_{(\text{Negative control group})} \times 100\%$$

wherein A was the reads from the enzyme-labeled instrument.

(3) Test results

The *in vitro* antiviral test showed that, $EC_{50}$ of the test compound BAY 2402234 for inhibiting the influenza viruses A/PR/8, A/California/07/2009, A/Hongkong/08/1968, A/Zhenxing1109/2010, and B/Lee/40 is 0.01±0.0003 μM, 0.03±0.009 μM, 0.007±0.003 μM, 0.04±0.01 μM, and 0.03±0.02 μM respectively (as shown in table 1); the cytotoxic test showed that, CC50 of the test compound on MDCK is 0.27±0.09 μM (as shown in table 1), and corresponding therapeutic index is 27, 9, 38.57, 6.75, and 9 respectively.

(4) Conclusion

The compound BAY 2402234 has relatively broad-spectrum inhibitory effect on influenza virus (H1N1, H1N1-Oseltamivir resistant strain, H3N2, type B).

Table 1 concentration for 50% maximal effect on influenza virus ($EC_{50}$) and safety ($CC_{50}$) of BAY 2402234

| Influenza virus (μM) | | | | | |
|---|---|---|---|---|---|
| PR8-MDCK | CA07-MDCK | HK68-MDCK | ZX1109-MDCK | Blee-MDCK | $CC_{50}$-MDCK |
| 0.01±0.0003 | 0.03±0.009 | 0.007±0.003 | 0.04±0.01 | 0.03±0.02 | 0.27±0.09 |

Example 3 *In vitro* evaluations of anti-zika virus activity and safety of BAY 2402234

(1) Evaluation of anti-zika virus activity of the drug

1) BHK cells (preserved in Academy of Military Medical Sciences) or Vero cells (preserved in Academy of Military Medical Sciences) were inoculated into a 96 wells plate at a concentration of $5\times10^3$ cells/well for BHK cells and $1\times10^4$ cells/well for Vero cells, wherein the medium is DMEM (purchased from Gibco, Catalog No.: 11995065) containing 10%FBS (purchased from Gibco, Catalog No.: 16000044), and cultured for 24 hrs at 37°C in a $CO_2$ incubator.

2) The culture medium in the 96-well plate was discarded, 100μL of DMEM medium (purchased from Gibco, Catalog No.:11995065) containing 2% FBS (purchased from Gibco, Catalog No.: 16000044) was added. The 50mM BAY 2402234 (purchased from MCE, Catalog No.: HY-112645) mother liquor was then diluted with the above DMEM medium in a serial manner to 800 nM, 266.67 nM, 88.89 nM, 29.63 nM, 9.88 nM, 3.29 nM, 1.10 nM, and 0.37 nM, 50 μL of each was added to the cell culture plate. Finally, 50μL of the zika virus strain SZ-SMGC-01 (preserved in Academy of Military Medical Sciences) diluted with DMEM medium containing 2% FBS was added to reach 100 $TCID_{50}$ for each well. The final concentration of the drug was 0.25 time to that of the drug before being treated, that is, the drug with 200 nM as initial concentration was diluted in 3-fold serial manner to reach final concentration of 200 nM, 66.67 nM, 22.22 nM, 7.41 nM, 2.47 nM, 0.82 nM, 0.27nM, and 0.09 nM. Negative control (wells only added with DMSO and the culture medium, without the drug) and Positive control (wells added with DMSO, culture medium and the virus, without the drug) were set up. The BHK cells were cultured at 37°C for 9 days, and the Vero cells were cultured for 7 days.

3) Buffer and substrate of CellTiter-Glo® Luminescent Cell Viability Assay Kit (purchased from Promega, Catalog No.: G7573) were mixed in dark as the work solution. The work solution and PBS (purchased from Gibco, Catalog No.: 10010049) were mixed in a ratio of 4:6. The liquid in the cell culture plate was discarded, and then 100μl test reagent was added for each well, the 96-well plate was shaked in an Orbital oscillator for 5min to induce cell lysis. After stablizing the signal in dark for 2min, chemiluminescence was determiend by an enzyme-labeled instrument (purchased from Molecular Devices, SpectraMax M5), the plate reading program was the CellTiter-Glo preset program, and the cell viability was calculated:

$$\text{cell viability (\%)} = (A_{(\text{drug treatment group})} - A_{(\text{Positive control group})}) / (A_{(\text{Negative control group})} - A_{(\text{Positive control group})}) \times 100\%$$

wherein A was the reads from the enzyme-labeled instrument.

(2) Cytotoxicity test of the drug

1) BHK cells (preserved in Academy of Military Medical Sciences) or Vero cells (preserved in Academy of Military Medical Sciences) were inoculated into a 96 wells plate at a concentration of $5\times10^3$ cells/well for BHK cells and $1\times10^4$ cells/well for Vero cells, wherein the medium is DMEM (purchased from Gibco, Catalog No.: 11995065) containing 10%FBS (purchased from Gibco, Catalog No.: 16000044), and cultured for 24 hrs at 37°C in a COz incubator.

2) The culture medium in the 96-well plate was discarded, 150μL DMEM medium (purchased from Gibco, Catalog

No.: 11995065) containing 2% FBS (purchased from Gibco, Catalog No.: 16000044) was added. The 50mM BAY 2402234 (purchased from MCE, Catalog No.: HY-112645) mother liquor was then diluted with the above DMEM medium in a serial manner to 800 nM, 266.67 nM, 88.89 nM, 29.63 nM, 9.88 nM, 3.29 nM, 1.10 nM, and 0.37 nM, 50 μL of each was added to the cell culture plate. The final concentration of the drug was 0.25 time to that of the drug before being treated, that is, the drug with 200 nM as initial concentration was diluted in 3-fold serial manner to reach final concentration of 200 nM, 66.67 nM, 22.22 nM, 7.41 nM, 2.47 nM, 0.82 nM, 0.27nM, and 0.09 nM. Negative control (wells only added with DMSO and the culture medium, without the drug) was set up. The BHK cells were cultured at 37°C for 9 days, and the Vero cells were cultured for 7 days.

3) Buffer and substrate of CellTiter-Glo® Luminescent Cell Viability Assay Kit (purchased from Promega, Catalog No.: G7573) were mixed in dark as the work solution. The work solution and PBS (purchased from Gibco, Catalog No.: 10010049) were mixed in a ratio of 4:6. The liquid in the cell culture plate was discarded, and then 100μl of test reagent was added for each well, the 96-well plate was shaked in an Orbital oscillator for 5min to induce cell lysis. After stablizing the signal in dark for 2min, chemiluminescence was determiend by an enzyme-labeled instrument (purchased from Molecular Devices, SpectraMax M5), the plate reading program was the CellTiter-Glo preset program, and the cytotoxicity was calculated:

$$\text{cytotoxicity (\%)} = (A_{(\text{Negative control group})} - A_{(\text{Drug treatment group})}) / A_{(\text{Negative control group})} \times 100\%$$

wherein A was the reads from the enzyme-labeled instrument.

(3) Test results

Preliminary test results showed that the test compound BAY 2402234 had inhibitory effect on zika virus.

Example 4: *In vitro* evaluations of anti-bunya virus activity and safety of BAY 2402234

(1) Evaluation of anti-bunya virus activity of the drug

1) Huh7 cells (preserved in Academy of Military Medical Sciences) were inoculated into a 96 wells plate at a concentration of $5\times10^3$ cells/well, wherein the medium is DMEM (purchased from Gibco, Catalog No.: 11995065) containing 10%FBS (purchased from Gibco, Catalog No.: 16000044), and cultured for 24 hrs at 37°C in a COz incubator.

2) The culture medium in the 96-well plate was discarded, 100μL DMEM medium (purchased from Gibco, Catalog No.:11995065) containing 2% FBS (purchased from Gibco, Catalog No.: 16000044) was added. The 50mM BAY 2402234 (purchased from MCE, Catalog No.: HY-112645) mother liquor was then diluted with the above DMEM medium in a serial manner to 800 nM, 266.67 nM, 88.89 nM, 29.63 nM, 9.88 nM, 3.29 nM, 1.10 nM, and 0.37 nM, 50 μL of each was added to the cell culture plate. Finally, 50μL of the bunya virus (isolated in the laboratory: from patient serum of Beijing Ditan Hospital Capital Medical University) diluted with DMEM medium containing 2% FBS was added to reach 100 $TCID_{50}$ for each well. The final concentration of the drug was 0.25 time to that of the drug before being treated, that is, the drug with 200 nM as initial concentration was diluted in 3-fold serial manner to reach final concentration of 200 nM, 66.67 nM, 22.22 nM, 7.41 nM, 2.47 nM, 0.82 nM, 0.27nM, and 0.09 nM. Negative control (wells only added with DMSO and the culture medium, without the drug) and Positive control (wells added with DMSO, culture medium and the virus, without the drug) were set up. The cells were cultured at 37°C for 6 days.

3) Buffer and substrate of CellTiter-Glo® Luminescent Cell Viability Assay Kit (purchased from Promega, Catalog No.: G7573) were mixed in dark as the work solution. The work solution and PBS (purchased from Gibco, Catalog No.: 10010049) were mixed in a ratio of 4:6. The liquid in the cell culture plate was discarded, and then 100μl test reagent was added for each well, the 96-well plate was shaked in an Orbital oscillator for 5min to induce cell lysis. After stablizing the signal in dark for 2min, chemiluminescence was determiend by an enzyme-labeled instrument (purchased from Molecular Devices, SpectraMax M5), the plate reading program was the CellTiter-Glo preset program, and the cell viability was calculated:

$$\text{cell viability (\%)} = (A_{(\text{drug treatment group})} - A_{(\text{Positive control group})}) / (A_{(\text{Negative control group})} - A_{(\text{Positive control group})}) \times 100\%$$

wherein A was the reads from the enzyme-labeled instrument.

(2) Cytotoxicity test of the drug

1) Huh7 cells (preserved in Academy of Military Medical Sciences) were inoculated into a 96 wells plate at a concentration of $5\times10^3$ cells/well, wherein the medium is DMEM (purchased from Gibco, Catalog No.: 11995065) containing 10%FBS (purchased from Gibco, Catalog No.: 16000044), and cultured for 24 hrs at 37°C in a COz incubator.

2) The culture medium in the 96-well plate was discarded, 150μL DMEM medium (purchased from Gibco, Catalog No.: 11995065) containing 2% FBS (purchased from Gibco, Catalog No.: 16000044) was added. The 50mM BAY 2402234 (purchased from MCE, Catalog No.: HY-112645) mother liquor was then diluted with the above DMEM medium in a serial manner to 800 nM, 266.67 nM, 88.89 nM, 29.63 nM, 9.88 nM, 3.29 nM, 1.10 nM, and 0.37 nM, 50 μL of each was added to the cell culture plate. The final concentration of the drug was 0.25 time to that of the drug before being treated, that is, the drug with 200 nM as initial concentration was diluted in 3-fold serial manner to reach final concentration of 200 nM, 66.67 nM, 22.22 nM, 7.41 nM, 2.47 nM, 0.82 nM, 0.27nM, and 0.09 nM. Negative control (wells only added with DMSO and the culture medium, without the drug) was set up. The cells were cultured at 37°C for 6 days.

3) Buffer and substrate of CellTiter-Glo® Luminescent Cell Viability Assay Kit (purchased from Promega, Catalog No.: G7573) were mixed in dark as the work solution. The work solution and PBS (purchased from Gibco, Catalog No.: 10010049) were mixed in a ratio of 4:6. The liquid in the cell culture plate was discarded, and then 100μl test reagent was added for each well, the 96-well plate was shaked in an Orbital oscillator for 5min to induce cell lysis. After stablizing the signal in dark for 2min, chemiluminescence was determiend by an enzyme-labeled instrument (purchased from Molecular Devices, SpectraMax M5), the plate reading program was the CellTiter-Glo preset program, and the cytotoxicity was calculated:

$$\text{cytotoxicity (\%)} = (A_{(\text{Negative control group})} - A_{(\text{Drug treatment group})}) / A_{(\text{Negative control group})} \times 100\%$$

wherein A was the reads from the enzyme-labeled instrument.

(3) Test results

Preliminary test results showed that the test compound BAY 2402234 had inhibitory effect on bunya virus.

Example 5 *In vitro* evaluations of anti-enterovirus activity and safety of BAY 2402234

(1) Evaluation of anti-enterovirus activity of the drug

1) Vero cells (purchased from ATCC, Catalog No.: CCL-81) were inoculated into a 96 wells plate at a concentration of $1\times10^4$ cells/well, wherein the medium is DMEM (purchased from Gibco, Catalog No.: 11995065) containing 2%FBS (purchased from Gibco, Catalog No.: 16000044), and cultured for 24 hrs at 37°C in a COz incubator.

2) Before the experiment, the cells were wahsed 3 times with PBS (purchased from Gibco, Catalog No.: 10010049), 100μL of DMEM medium (purchased from Gibco, Catalog No.:11995065) containing 2% FBS (purchased from Gibco, Catalog No.: 16000044) was added. The 50mM BAY 2402234 (purchased from MCE, Catalog No.: HY-112645) mother liquor was then diluted with the above DMEM medium in a serial manner to 40 μM, 13.33 μM, 4.44 μM, 1.48 μM, 0.49 μM, 0.16 μM, 0.05 μM, 18.29 nM, 6.09 nM, and 2.03 nM, 50 μL of each was added to the cell culture plate. Finally, 50μL of the enterovirus strain CB3 (preserved in Academy of Military Medical Sciences) diluted

with DMEM medium containing 2% FBS was added to reach 100 $TCID_{50}$ for each well. The final concentration of the drug was 0.25 time to that of the drug before being treated, that is, the drug with 10 μM as initial concentration was diluted in 3-fold serial manner to reach final concentration of 10 μM, 3.33 μM, 1.11 μM, 0.37μM, 0.12μM, 0.04μM, 13.72nM, 4.57nM, 1.52nM, and 0.51nM. Negative control (wells only added with DMSO and the culture medium, without the drug) and Positive control (wells added with DMSO, culture medium and the virus, without the drug) were set up. The cells were cultured at 37°C for 5 days.

3) Buffer and substrate of CellTiter-Glo® Luminescent Cell Viability Assay Kit (purchased from Promega, Catalog No.: G7573) were mixed in dark as the work solution. The work solution and PBS were mixed in a ratio of 4:6. The liquid in the cell culture plate was discarded, and then 100μl of test reagent was added for each well, the 96-well plate was shaked in an Orbital oscillator for 5min to induce cell lysis. After stablizing the signal in dark for 3min, chemiluminescence was determiend by an enzyme-labeled instrument (purchased from Molecular Devices, SpectraMax M5), the plate reading program was the CellTiter-Glo preset program, and the cell viability was calculated:

$$\text{cell viability (\%)} = (A_{\text{(drug treatment group)}} - A_{\text{(Positive control group)}}) / (A_{\text{(Negative control group)}} - A_{\text{(Positive control group)}}) \times 100\%$$

wherein A was the reads from the enzyme-labeled instrument.

(2) Cytotoxicity test of the drug

1) Vero cells (purchased from ATCC, Catalog No.: CCL-81) were inoculated into a 96 wells plate at a concentration of $1\times10^4$ cells/well, wherein the medium is DMEM (purchased from Gibco, Catalog No.: 11995065) containing 10%FBS (purchased from Gibco, Catalog No.: 16000044), and cultured for 24 hrs at 37°C in a COz incubator.

2) Before the experiment, the cells were wahsed 3 times with PBS (purchased from Gibco, Catalog No.: 10010049), 150μL of DMEM medium (purchased from Gibco, Catalog No.:11995065) containing 2% FBS (purchased from Gibco, Catalog No.: 16000044) was added. The 50mM BAY 2402234 (purchased from MCE, Catalog No.: HY-112645) mother liquor was then diluted with the above DMEM medium in a serial manner to 40 μM, 13.33 μM, 4.44 μM, 1.48 μM, 0.49 μM, 0.16 μM, 0.05 μM, 18.29 nM, 6.09 nM, and 2.03 nM, 50 μL of each was added to the cell culture plate. The final concentration of the drug was 0.25 time to that of the drug before being treated, that is, the drug with 10 μM as initial concentration was diluted in 3-fold serial manner to reach final concentration of 10 μM, 3.33 μM, 1.11 μM, 0.37μM, 0.12μM, 0.04μM, 13.72nM, 4.57nM, 1.52nM, and 0.51nM. Negative control (wells only added with DMSO and the culture medium, without the drug) was set up. The cells were cultured at 37°C for 5 days.

3) Buffer and substrate of CellTiter-Glo® Luminescent Cell Viability Assay Kit (purchased from Promega, Catalog No.: G7573) were mixed in dark as the work solution. The work solution and PBS were mixed in a ratio of 4:6. The liquid in the cell culture plate was discarded, and then 100μl of test reagent was added for each well, the 96-well plate was shaked in an Orbital oscillator for 5min to induce cell lysis. After stablizing the signal in dark for 3min, chemiluminescence was determiend by an enzyme-labeled instrument (purchased from Molecular Devices, SpectraMax M5), the plate reading program was the CellTiter-Glo preset program, and the cytotoxicity was calculated:

$$\text{cytotoxicity (\%)} = (A_{\text{(Negative control group)}} - A_{\text{(Drug treatment group)}}) / A_{\text{(Negative control group)}} \times 100\%$$

wherein A was the reads from the enzyme-labeled instrument.

(3) Test results

Preliminary test results showed that the test compound BAY 2402234 had inhibitory effect on enterovirus.

| Enterovirus (μM) | | | | | |
|---|---|---|---|---|---|
| EV71-RD | EVD68-RD | CA16-RD | CA6-RD | CB3-Vero | $CC_{50}$ |
| >100 | >100 | >100 | >100 | <0.05 | >300 |

In addition to those described herein, according to the above descriptions, various modifications of the invention will be obvious for a person skilled in the art. Such modifications also fall within the scope of the appended claims. Each of the references (including all patents, patent applications, journal articles, books, and any other publications) cited in this application is hereby incorporated by reference in its entirety.

## Claims

**1.** Use of a compound of formula I, or its N-oxide, tautomer, geometric isomer, solvate, hydrate, pharmaceutically acceptable salt, or pharmaceutically acceptable salt of the tautomer or N-oxide thereof in the manufacture of a medicament, wherein the medicament is used in preventing and/or treating a disease associated with a virus, and the compound of formula I is as shown below,

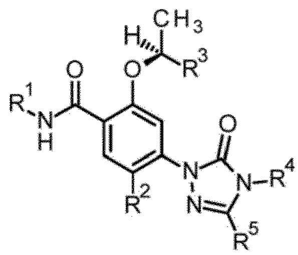

I

wherein,

$R^1$ represents a group selected from
3-pentyl, 2,2-dimethylpropyl, 4-heptyl, 4-fluorophenylcyclopropyl, cyclopentyl, cyclohexyl, cycloheptyl, cyclopentylmethyl, cyclohexylmethyl, 1-cyclohexylethyl, 1-hydroxypropan-2-yl, 2-hydroxypropyl, 1-hydroxybutan-2-yl, 1-cyanobutan-2-yl, 1-phenylbutan-2-yl, 1-amino-2-propyl, 1-amino-2-butyl, 1-amino-1-oxobutan-2-yl, indan-2-yl,
a 5- to 6-membered heterocycloalkyl group, which is selected from tetrahydrofuran-3-yl, tetrahydro-2H-pyran-4-yl and piperidin-4-yl, and which is optionally substituted one or two times with a methyl group,
a phenyl group, which is optionally substituted, one, two or three times, each substituent is independently selected from a fluorine atom or a chlorine atom or a group selected from methyl, ethyl, propyl, isopropyl, difluoromethyl, trifluoromethyl, methoxy, -O-C(=O)-1,1-dimethylethyl, hydroxy, $-C(=O)OCH_3$, -C(=O)NH-cyclopropyl, amino, methylamino, aminomethyl, $-S-CH_3$, $-S(=O)_2CH_3$, and $-S(=O)(NH)CH_3$, and
a monocyclic heteroaryl group, which is selected from oxazol-2-yl, pyrazol-3-yl, pyrazol-5-yl, pyridin-2-yl, pyridin-3-yl, pyridin-4-yl, pyrimidin-2-yl, pyrimidin-4-yl, chinolin-5-yl, indazol-5-yl, and which is optionally substituted one or two times, each substituent is independently selected from methyl and methoxy,
$R^2$ represents a hydrogen atom or a fluorine or chlorine atom,
$R^3$ represents a group selected from propyl, 2-methylpropyl, 3-pentyl, cyclopropylmethyl, cyclopropyl, cyclopropylmethyl, cyclobutyl, cyclopentyl, cyclohexyl, difluoromethyl, trifluoromethyl, 1,1-difluoroethyl, prop-2-en-1-yl, 2-methyl-prop-1-en-1-yl, N,N-dimethylaminoethyl, and phenyl,
$R^4$ represents a group selected from methyl, ethyl, propyl, isopropyl, 2-butyl, prop-2-en-1-yl, cyclopropylmethyl, benzyl, cyclopropyl, cyclobutyl, cyclopentyl, and 2-hydroxyethyl,
$R^5$ represents a chlorine atom or a group selected from methyl, ethyl, propyl, isopropyl, 2-butyl, isobutyl, tert-butyl, cyclopropyl, cyclobutyl, cyclopentyl, trifluoromethyl, hydroxymethyl, 1-hydroxyethyl, 2-hydroxypropan-2-yl, 1-chloroethyl, 1-hydroxy-2,2,2-trifluoroethyl, 1-methoxyethyl, methoxy, isopropoxy, methylsulfanyl, aminomethyl, (methylamino)methyl, (dimethylamino)methyl, 1-aminoethyl, 2-aminoethyl, methylamino and ethyl(methyl)amino, -C(=O)OH, $-C(=O)OCH_3$, $-C(=O)NH_2$, $-C(=O)NHCH_3$, -C(=O)NHcyclopropyl,

$-C(=O)N(CH_3)_2$, and $-S(=O)(=NH)CH_3$.

**2.** Use according to claim 1, wherein the compound is the compound of formula II,

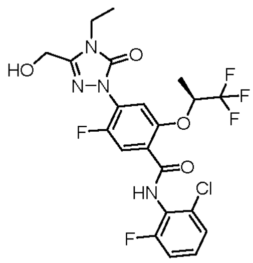

II.

**3.** Use of the compound as defined in claim 1 or 2, or its N-oxide, tautomer, geometric isomer, solvate, hydrate, pharmaceutically acceptable salt, or pharmaceutically acceptable salt of the tautomer or N-oxide thereof in the manufacture of a medicament, wherein the medicament is used to inhibit the replication or reproduction of a virus in a cell (e.g., a mammal cell).

**4.** Use according to any one of claims 1-3, wherein the virus is a RNA virus.

**5.** Use according to any one of claims 1-4, wherein the virus is a Coronaviridae virus, an Orthomyxoviridae virus, a Flaviviridae virus, a Bunyaviridae virus, a Picornaviridae virus, an Arenavirus, a Filoviridae virus, or a WEE virus;

preferably, the Coronaviridae virus is selected from HCoV-229E, HCoV-OC43, HCoV-NL63, HCoV-HKU1, SARS-CoV, MERS-CoV, and SARS-CoV-2;
preferably, the Orthomyxoviridae virus is an Influenza virus, such as Influenza A virus, Influenza B virus, or Influenza C virus;
preferably, the Flaviviridae virus is selected from Zika virus, Dengue virus, West Nile virus, Yellow fever virus and HCV;
preferably, the Bunyaviridae virus is a Bunya virus or Phlebovirus;
preferably, the Picornaviridae virus is Enterovirus or Foot-and-Mouth Disease virus;
preferably, the Filoviridae virus is selected from Ebola virus, Marburg virus, and Cueva virus.

**6.** Use according to any one of claims 1-5, wherein the medicament is used to prevent and/or treat a disease caused by SARS-CoV, MERS-CoV, SARS-CoV-2, Influenza virus, Zika virus, Dengue virus, Bunya virus, or Enterovirus;

preferably, the medicament is used to prevent and/or treat a disease caused by SARS-CoV, MERS-CoV, or SARS-CoV-2;
preferably, the medicament is used to prevent and/or treat a disease caused by SARS-CoV-2, for example, simple infection (such as, fever, cough, and/or pharyngalgia), pneumonia, acute or severe respiratory infection, hypoxic respiratory failure, acute respiratory distress syndrome, sepsis or septic shock;
preferably, the medicament is used to prevent and/or treat COVID-19.

**7.** Use according to any one of claims 1-6, wherein the medicament is for human or animal use.

**8.** Use according to any one of claims 1-7, wherein the medicament further comprises a pharmaceutically acceptable carrier or excipient.

**9.** Use according to any one of claims 1-8, wherein the compound is the only pharmaceutical active ingredient, or is used in combination with other pharmaceutical active ingredients (e.g., the medicament is a compound preparation); preferably, the other pharmaceutical active ingredients are antiviral drugs, such as one or more selected from amantadine, rimantadine, enfuvirtide, maraviroc, acyclovir, ganciclovir, valaciclovir, famciclovir, sodium phosphonate, lamivudine, zidovudine, enteltabine, tenofovir, adefovir dipivoxil, efavirenz, nevirapine, saquinavir, oseltamivir, zanamivir, ribavirin, and interferon.

**10.** Use according to any one of claims 1-9, wherein the medicament is a solid preparation or a liquid preparation; preferably, the medicament is a tablet, an injection, or a spray; preferably a tablet or injection.

**11.** A method of preventing and/or treating a disease associated with a virus, comprising a step of administering to a subject in need an effective amount of the compound as defined in claim 1 or 2, or its N-oxide, tautomer, geometric isomer, solvate, hydrate, pharmaceutically acceptable salt, or pharmaceutically acceptable salt of the tautomer or N-oxide thereof.

**12.** A method of inhibiting the replication or reproduction of a virus in a cell (e.g., a mammal cell), comprising a step of administering to a subject in need or contacting the cell with an effective amount of the compound as defined in claim 1 or 2, or its N-oxide, tautomer, geometric isomer, solvate, hydrate, pharmaceutically acceptable salt, or pharmaceutically acceptable salt of the tautomer or N-oxide thereof.

**13.** The method according to claim 11 or 12, wherein the virus is a RNA virus.

**14.** The method according to any one of claims 11-13, wherein the virus is a Coronaviridae virus, an Orthomyxoviridae virus, a Flaviviridae virus, a Bunyaviridae virus, a Picornaviridae virus, an Arenavirus, a Filoviridae virus, or a WEE virus;

preferably, the Coronaviridae virus is selected from HCoV-229E, HCoV-OC43, HCoV-NL63, HCoV-HKU1, SARS-CoV, MERS-CoV, and SARS-CoV-2;
preferably, the Orthomyxoviridae virus is an Influenza virus, such as Influenza A virus, Influenza B virus, or Influenza C virus;
preferably, the Flaviviridae virus is selected from Zika virus, Dengue virus, West Nile virus, Yellow fever virus and HCV;
preferably, the Bunyaviridae virus is a Bunya virus or Phlebovirus;
preferably, the Picornaviridae virus is Enterovirus or Foot-and-Mouth Disease virus;
preferably, the Filoviridae virus is selected from Ebola virus, Marburg virus, and Cueva virus.

**15.** The method according to any one of claims 11-14, wherein the compound, or its N-oxide, tautomer, geometric isomer, solvate, hydrate, pharmaceutically acceptable salt, or pharmaceutically acceptable salt of the tautomer or N-oxide thereof is used to prevent and/or treat a disease caused by SARS-CoV, MERS-CoV, SARS-CoV-2, Influenza virus, Zika virus, Dengue virus, Bunya virus, or Enterovirus;

preferably, the compound, or its N-oxide, tautomer, geometric isomer, solvate, hydrate, pharmaceutically acceptable salt, or pharmaceutically acceptable salt of the tautomer or N-oxide thereof is used to prevent and/or treat a disease caused by SARS-CoV, MERS-CoV, or SARS-CoV-2;
preferably, the compound, or its N-oxide, tautomer, geometric isomer, solvate, hydrate, pharmaceutically acceptable salt, or pharmaceutically acceptable salt of the tautomer or N-oxide thereof is used to prevent and/or treat a disease caused by SARS-CoV-2, for example, simple infection (such as, fever, cough, and/or pharyngalgia, pneumonia, acute or severe respiratory infection, hypoxic respiratory failure, acute respiratory distress syndrome, sepsis or septic shock;
preferably, the compound, or its N-oxide, tautomer, geometric isomer, solvate, hydrate, pharmaceutically acceptable salt, or pharmaceutically acceptable salt of the tautomer or N-oxide thereof is used to prevent and/or treat COVID-19.

**16.** The method according to any one of claims 11-15, wherein the subject is a mammal, for example, human.

**17.** The method according to claim 12, wherein the mammal cell is a human cell.

**18.** The method according to any one of claims 11-17, wherein the compound, or its N-oxide, tautomer, geometric isomer, solvate, hydrate, pharmaceutically acceptable salt, or pharmaceutically acceptable salt of the tautomer or N-oxide thereof is administered only or in combination with other pharmaceutical active ingredients, for example, administered concurrently, separately, or successively;
preferably, the other pharmaceutical active ingredients are antiviral drugs, such as one or more selected from amantadine, rimantadine, enfuvirtide, maraviroc, acyclovir, ganciclovir, valaciclovir, famciclovir, sodium phosphonate, lamivudine, zidovudine, enteltabine, tenofovir, adefovir dipivoxil, efavirenz, nevirapine, saquinavir, oseltamivir, zanamivir, ribavirin, and interferon.

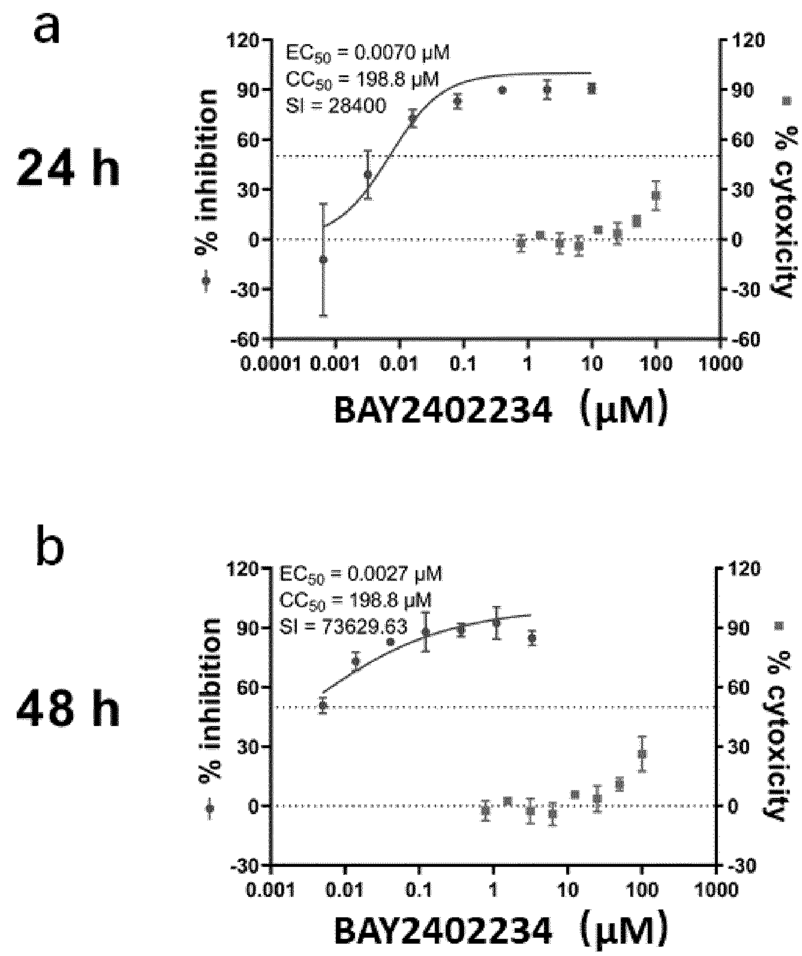
a
24 h
EC50 = 0.0070 μM
CC50 = 198.8 μM
SI = 28400
% inhibition
% cytoxicity
BAY2402234 (μM)
b
48 h
EC50 = 0.0027 μM
CC50 = 198.8 μM
SI = 73629.63
% inhibition
% cytoxicity
BAY2402234 (μM)

Fig. 1

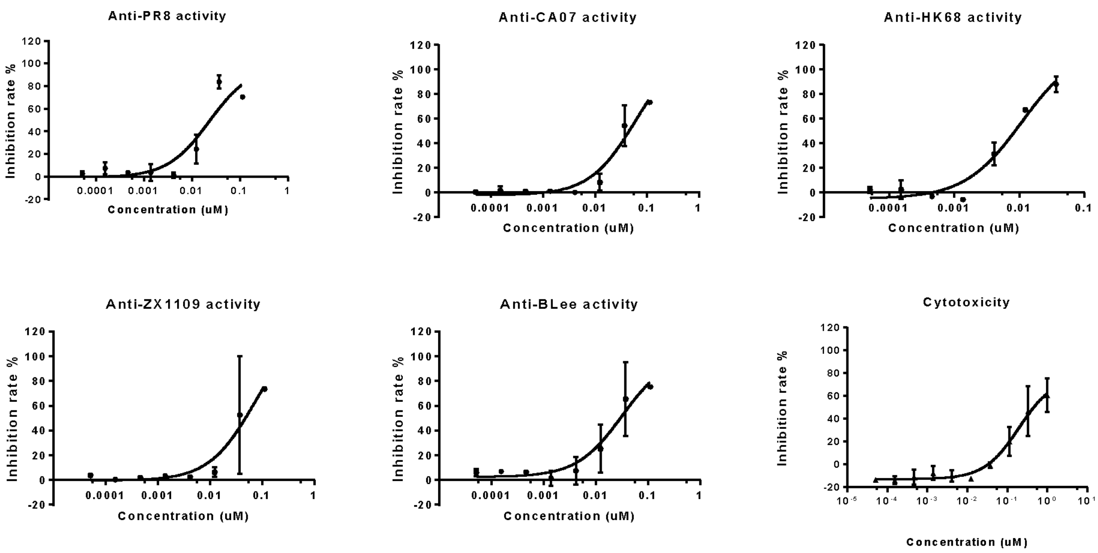
Anti-PR8 activity
Anti-CA07 activity
Anti-HK68 activity
Anti-ZX1109 activity
Anti-BLee activity
Cytotoxicity
Inhibition rate %
Concentration (uM)

Fig. 2

**INTERNATIONAL SEARCH REPORT**

International application No. **PCT/CN2021/106486**

**A. CLASSIFICATION OF SUBJECT MATTER**

A61K 31/4196(2006.01)i； A61P 31/14(2006.01)i； A61P 31/16(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS; CNTXT; DWPI; SIPOABS; WOTXT; USTXT; EPTXT; CNKI; 万方; 读秀学术; Springer; ISI Web of Science: 中国人民解放军军事科学院军事医学研究院, 钟武, 周辛波, 曹瑞源, 肖典, 王曼丽, 樊士勇, 胡志红, 李松, 2, 4, 5-三取代的1, 2, 4-三唑酮, 二氢乳清酸脱氢酶, 抑制剂, RNA病毒, 寨卡病毒, 新型冠状病毒, BAY2402234, Academy of Military Medical Sciences, ZHONG Wu, ZHOU Xinbo, CAO Ruiyuan, XIAO Dian, WANG Manli, FAN Shiyong, HU Zhihong, LI Song, 2, 4, 5-trisubstituted-1, 2, 4-triazolone, dihydroorotate dehydrogenase, DHODH, inhibitor, RNA viruse, Zika Virus, COVID-19, SARS-CoV-2

**C. DOCUMENTS CONSIDERED TO BE RELEVANT**

| Category* | Citation of document, with indication, where appropriate, of the relevant passages | Relevant to claim No. |
|---|---|---|
| PX | Kim M. Stegmann et al. "N4-hydroxycytidine and Inhibitors of Dihydroorotate Dehydrogenase Synergistically Suppress SARS-CoV-2 Replication" *BioRxiv*, 28 June 2021 (2021-06-28), pages 1-30, in particular page 10, paragraph 2 | 1-10 |
| PX | CN 111773214 A (ACAD OF MILITARY SCIENCES PLA CHINA ACAD OF MILITARY MEDICAL SCIENCES) 16 October 2020 (2020-10-16) claims 1-10 | 1-10 |
| X | CN 110023302 A (BAYER AG et al.) 16 July 2019 (2019-07-16) description paragraphs [0015-0017], [0189], [1753], [1754], [3629], [3631], [3670], [3673], [3705] | 1-3, 7-10 |
| Y | CN 110023302 A (BAYER AG et al.) 16 July 2019 (2019-07-16) description paragraphs [0015-0017], [0189], [1753], [1754], [3629], [3631], [3670], [3673], [3705] | 4-10 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 September 2021** | **27 September 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/106486**

**C. DOCUMENTS CONSIDERED TO BE RELEVANT**

| Category* | Citation of document, with indication, where appropriate, of the relevant passages | Relevant to claim No. |
|---|---|---|
| Y | XIONG, Rui et al. "Novel and Potent Inhibitors Targeting DHODH, a Rate-limiting Enzyme in de Novo Pyrimidine Biosynthesis, Are Broad-spectrum Antiviral Against RNA Viruses Including Newly Emerged Coronavirus SARS-CoV-2" *BioRxiv*, 12 March 2020 (2020-03-12), pages 1-31, in particular abstract, page 12, last paragraph, page 13, paragraph 1 | 4-10 |
| A | None. "华理与武大团队联合发现强效抗新冠病毒候选药物和老药品种 (Non-official translation: East China University of Science and Technology and Wuhan University Have Jointly Discovered An Effective Anti-COVID-19 Drug Candidate and A Drug Currently Used)" *上海化工 (Shanghai Chemical Industry)*, Vol. 45, No. 2, 30 April 2020 (2020-04-30), p. 76 | 1-10 |

Form PCT/ISA/210 (second sheet) (January 2015)

**INTERNATIONAL SEARCH REPORT**

International application No.
**PCT/CN2021/106486**

**Box No. II Observations where certain claims were found unsearchable (Continuation of item 2 of first sheet)**

This international search report has not been established in respect of certain claims under Article 17(2)(a) for the following reasons:

1. [✓] Claims Nos.: **11-18**
because they relate to subject matter not required to be searched by this Authority, namely:

[1] The subject matter of claim 11 relates to a method for preventing and/or treating diseases associated with virus, and the subject matter of claim 12 relates to a method for inhibiting virus replication and propagation in cells. However, the ultimate purpose of the claimed methods is to treat diseases in the living animal body, which falls within the scope of methods for diagnosing and treating diseases under PCT Rule 39.1(4).

[2] Based on the same reason, dependent claims 13-18 also pertain to methods for diagnosing and treating diseases under PCT Rule 39.1(4).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/106486**

| Patent document cited in search report | Publication date (day/month/year) | Patent family member(s) | Publication date (day/month/year) |
|---|---|---|---|
| CN 111773214 A | 16 October 2020 | CN 111773214 B | 20 April 2021 |
| CN 110023302 A | 16 July 2019 | US 2020123129 A1 | 23 April 2020 |
| | | US 10815215 B2 | 27 October 2020 |
| | | JP 2019533694 A | 21 November 2019 |
| | | KR 20190084954 A | 17 July 2019 |
| | | US 2020377472 A1 | 03 December 2020 |
| | | WO 2018077923 A1 | 03 May 2018 |
| | | MX 2019005009 A | 12 August 2019 |
| | | AU 2017351688 A1 | 11 April 2019 |
| | | CA 3041643 A1 | 03 May 2018 |
| | | BR 112019008458 A2 | 09 July 2019 |
| | | EP 3532468 A1 | 04 September 2019 |
| | | TW 201827414 A | 01 August 2018 |
| | | US 20210188805 A9 | 24 June 2021 |
| | | SG 11201902392 A1 | 29 April 2019 |
| | | HK 40006139 A0 | 15 May 2020 |
| | | VN 64781 A | 25 July 2019 |
| | | IN 201917015049 A | 19 July 2019 |
| | | ID 201907156 A | 27 September 2019 |
| | | PH 12019500932 A1 | 20 January 2020 |
| | | GE 202107248 B | 26 April 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010692337


### Non-patent literature cited in the description


- **T. L. GILCHRIST.** Comprehensive Organic Synthesis. Academic Press, vol. 7, 748-750
- **G. W. H. CHEESEMAN ; E. S. G. WERSTIUK.** Advances in Heterocyclic Chemistry. Academic Press, vol. 22, 390-392